# EUROPEAN PATENT APPLICATION

(11) **EP 1 532 989 A1**
(43) Date of publication of application: **25.05.2005**
(21) Application number: 04256619.0
(22) Date of filing: 01.11.2004
(51) Int. Cl.: A61L 2/10

(54) **Medical ultrasound transducer UV sterilization device**

(30) Priority: 10.11.2003 US 518951 P
(71) Applicant: Sonotech, Inc., Bellingham, WA 98225 (US)
(72) Inventor: Larson, Eugene A., Lummi Island, WA 98262 (US); Larson, Margaret J., Lummi Island, WA 98262 (US)
(74) Representative: Naylor, Matthew John

(57) **Abstract**

An apparatus for delivering a sterilizing dose of ultraviolet wavelength (UV) light to sterilize one or more patient contacting surfaces of a transducer (4). The dose of ultraviolet light can be delivered to the face and, preferably, also to a portion of the sides of the transducer area, which is the patient contact area, in a short period of time.

## Description

### Field of the Invention

The present invention is directed to the sterilization of medical instruments, particularly but not exclusively to the sterilization of ultrasound transducers or probes, utilizing ultraviolet wavelength (UV) light.

### Background of the Invention

Ultrasound has been used widely since the 1970's as a non-invasive modality for, among other things, the diagnosis and therapy of human disease, and for the determination and monitoring of pregnancy and the status of a fetus.

Ultrasound imaging and therapy is interfaced with the patient by a transducer, which is in contact with the patient (usually via a liquid, gel and/or solid membrane ultrasound couplant) and converts electrical signals into sound waves and the returning sound echoes back into electrical signals. This transducer, also referred to as a probe or scan head, is expensive to manufacture and purchase, and is made available in many frequencies and configurations to cover a broad range of imaging and therapy applications.

Most users of medical ultrasound electronics for imaging and therapy have more than one transducer of a specific type for each system in use. These transducers are used repeatedly from patient to patient in a manner that is in contact with and scans across each patient's skin.

Over the past 20 years, bacteria, virus and fungus transmitted from patient to patient have become a growing concern. More patients are immune compromised from disease (e.g. AIDS) and from chemotherapy (e.g. cancer treatment). Bacteria and virus have emerged that are easily transmitted and highly resistant to available drugs (such as, for example, drug resistant Staphylococcus, and Tuberculosis).

Of particular concern is methicillin-resistant Staphylococcus aureus (MRSA), now a common hospital infection. MRSA now accounts for over 30% of all hospital staph cultures, up from less than 10% in 1997. While most strains of MRSA require a break in the skin as a foothold for infection, the newer strains appear to produce a toxin that can break through intact skin.

The US Center for Disease Control and Prevention (CDC) estimates that roughly 15% of the MRSA infections are now "community acquired" among people who have not been hospitalized. This brings a new level of infection risk and nosocomial infection associated with diagnostic and therapeutic ultrasound procedures.

Cleaning and sterilization of ultrasound transducers between patients is difficult and time consuming. A current method consists of a cold sterilization process comprising soaking a transducer in a sterilizing solution for 30 minutes. The number of imaging or therapy procedures performed using an ultrasound transducer leaves little time (e.g. minutes) for cleaning the transducer before the next use.

Sterilizing (killing pathogens that may cause human disease) an ultrasound probe or transducer between patients is further complicated by the materials used in the construction of the device. Autoclaving is not possible due to heat damage. Soaking the transducer in disinfecting solution is too time consuming and the harsh chemicals may damage the lens of the transducer, ultimately causing ground fault failure of the unit. While ultrasound probe covers, typically made of latex or polyurethane, that may be discarded after a single use are known, they may tear or rupture during the examination thus exposing the patient to the non sterile contaminated surface of the transducer. Other drawbacks or disadvantages of the ultrasound probe covers Include the generation of significant waste, added cost and time to the examination, and often the degradation of the ultrasound image during the examination.

### Summary of the Invention

The present invention delivers a controlled sterilizing dose of ultraviolet wavelength (UV) light to sterilize one or more patient contacting surfaces of a transducer. The dose of ultraviolet light can be delivered to the face and, preferably, also to a portion of the sides of the transducer area, which is the patient contact area, in a short period of time.

Aspects of the present invention comprise an enclosure that may be mounted on a wall, exam table, on the ultrasound instrument or incorporated into an ultrasound system. The enclosure retains the transducer, distributes the ultraviolet light over the transducer face and immediate front sides of the transducer, and protects the patient and technician from ultraviolet exposure.

The aspects of the invention may include a timer that insures the duration of a dose of UV required for sterilization (e.g. about five minutes), a UV output monitor (to constantly monitor the lamp output and provide a signal for bulb replacement) and/or an electronic device that measures the length of time bulbs have been in service and signals or disables the device when bulb replacement is required.

In addition to the sterilization of ultrasound transducers used for imaging, aspect of the present invention also contemplate the sterilization of ultrasound transducers used for therapeutic procedures such as in physical therapy and High Intensity Focused Ultrasound (HIFU) therapy to sterilize the transducer between procedures. The ultraviolet coverage of the transducer can be extended to cover the entire transducer, including very long transducers, such as transesophageal and endoscopic, by extending the length of the enclosure, increasing the number of UV bulbs and/or increasing the number or area of the reflective surfaces.

According to one aspect of the present invention there is provided an apparatus for sterilizing one or more surfaces of an ultrasound transducer, said apparatus comprising:
a chamber comprising an opening through which an ultrasound transducer can be inserted into and withdrawn from the chamber;
at least one source of UV light capable of delivering a dose of ultraviolet wavelength (UV) light into said chamber, said dose being sufficient to sterilize one or more surfaces of the transducer.

Said chamber may include one or more reflecting surfaces and said one or more reflecting surfaces may comprise mirrors. Said mirrors may comprise one or both of coated metals and metalized plastics.

Said source of UV light may comprise a UV lamp.

Said chamber may include means to secure in a releasable manner the one or more surfaces of the transducer within said chamber.

The apparatus may further include a movable tray for transporting an ultrasound transducer toward and away from said chamber whereby the one or more surfaces of the transducer may be inserted into and withdrawn from said chamber via said opening.

The apparatus may further include a programmable or fixed exposure timer for said source of UV light.

The apparatus may further include a UV output monitor. Said output monitor may include a signal means for indicating the need to replace the source of UV light.

The apparatus may include means to measure the length of time the source of UV light has been in service. Alternatively or additionally, the apparatus may further include a means to provide a signal indicative of when the UV light source has been in service for a predetermined amount of time. Alternatively or additionally, the apparatus may further include a means to disable said apparatus when the UV light source has been in service for a predetermined amount of time.

Said chamber may be located within an outer enclosure.

The apparatus may include mounting surfaces whereby said apparatus is positionable on a generally horizontal surface. Alternatively or additionally, the apparatus may include means to mount said apparatus on a generally vertical surface. The apparatus may be positionable in a medical ultrasound system, an electronic system or a control cabinet.

Another aspect of the present invention provides a method of sterilizing one or more surfaces of an ultrasound transducer, said method comprising:
providing a chamber comprising an opening through which the ultrasound transducer can be inserted into and withdrawn from the chamber, said chamber including a source of UV light capable of delivering a dose of ultraviolet wavelength (UV) light into said chamber;
inserting the one or more surfaces of the ultrasound transducer into said chamber;
activating the UV light and exposing said one or more surfaces of the transducer to said UV light for a predetermined amount of time whereby said one or more surfaces of the transducer are sterilized;
withdrawing the sterilized transducer from the chamber.

Said chamber may include one or more reflecting surfaces.

Said one or more surfaces of the transducer may comprise a front face and front sides of the transducer and the front face of the transducer may include a UV inhibitor.

Said exposure may be at least at a UV wavelength of 253.7 nanometer and a dose of 300,000 microwatt seconds per square centimeter for 5 minutes.

The method may further comprise cleaning said one or more surfaces of the ultrasound transducer prior to said exposure.

Said ultrasound transducer may be one of an ultrasound transducer utilized for imaging procedures, an ultrasound transducer utilized for therapeutic procedures, or, a high intensity focused ultrasound (HIFU) transducer.

### Brief Description of the Drawings

Figures 1 (a), 1 (b) and 1 (c) illustrate respective front, side and top views of a sterilizing enclosure in accordance with a first embodiment.

Figures 2(a), 2(b) and 2(c) illustrate respective front, side and top views of a sterilizing enclosure in accordance with a second embodiment.

### Detailed Description of the Preferred Embodiments

The present invention will now be discussed with reference to preferred embodiments and the drawings which represent the invention by way of example only. In the drawings, like features will be referred to by the same reference number.

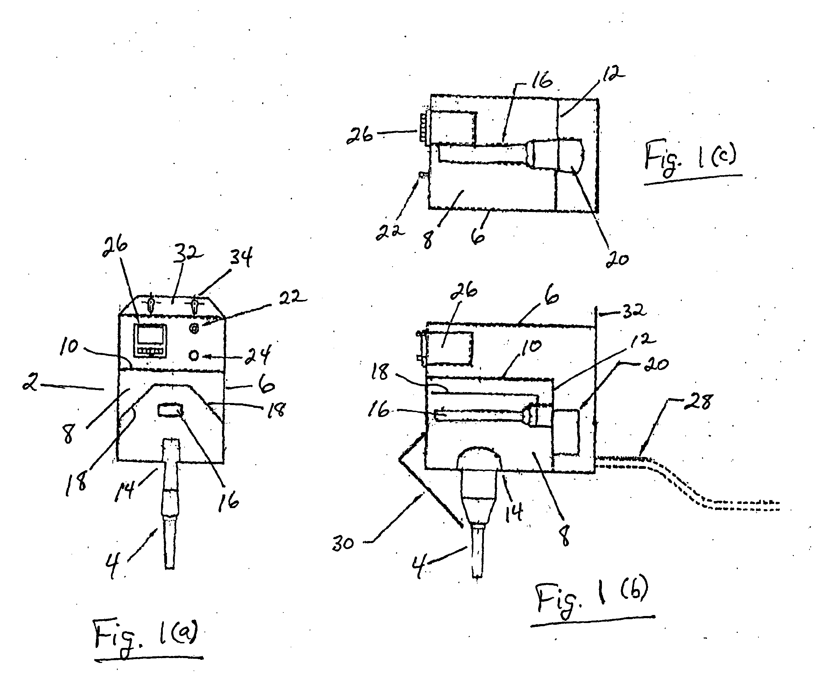

A first embodiment of the present invention is exemplified in Figures 1 (a)-(c) wherein an enclosure 2 for sterilizing a diagnostic or therapeutic ultrasound transducer 4 is illustrated. The enclosure 2 comprises an outer shell portion 6 made of a suitable material, such as stainless steel, aluminum or plastic. Located within the outer shell portion 6 is an inner chamber 8 defined by inner partitions 10 and 12 made of, for example, stainless steel, into which transducer 4 may be inserted through an opening 14 in the outer shell 6. An ultraviolet lamp 16 (for example, a 9 watt UV lamp) and one or more reflecting surfaces 18 (e.g. mirrors, such as those being highly polished or of coated metals or metalized plastics) are located within inner chamber 8. If necessary for operation of the lamp 16, a ballast 20 may be included within shell portion 6.

Located on an outside surface of shell 6, for example, the front surface as shown in Figure 1 (a), is a power on/off switch 22, a cycle start button 24 and a programmable or fixed exposure timer 26. Power is supplied to the enclosure 2 via power cord 28. Outer shell portion 6 may also include a hinged cover 30 (Figure 1(b)) which is swung open to allow insertion of transducer 4 through opening 14 and into a releasable secured position within the inner chamber 8. In the above arrangement, the bottom of the hinged cover 30 preferably includes a U-shaped slot into which the transducer handle is inserted upon closing of the hinged cover 30 thereby preventing UV light from exiting the enclosure and creating an exposure hazard. Alternatively, transducer 4 may be held in position in the inner chamber 8 via a clip (not shown) that would grip the smaller diameter handle of the transducer.

The enclosure 2 may include a flanged portion 32 with one or more openings 34 to permit mounting or hanging the enclosure 2 on, for example, a wall or on the ultrasound instrument surface, via suitable means such as screws, nails and the like. If desired, an ultrasound instrument may have a recessed area formed in its outer surface for receiving an enclosure such as shown in Figures 1(a)-(c) with or without the flanged portion 32. The enclosure may also be received in an electronic module or control cabinet. The enclosure 2 is preferably compact in size with typical dimensions being, with reference to Figure 1(a), a width of 6 inches and a height of 10 inches, and, with reference to Figure 1(c), a length or depth of 9 inches. Of course, the inventive enclosure is not to be limited by any particular size or dimension. The enclosure 2 should be appropriately sized as is necessary to accommodate the particular ultrasound transducer or range of different size transducers.

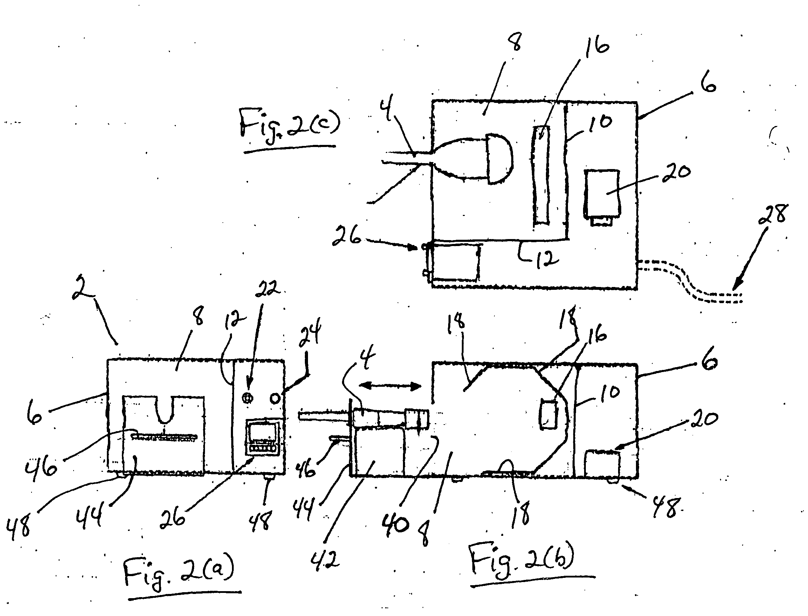

Figures 2 (a)-(c) illustrate another enclosure 2 suitable for resting on a horizontal surface such as a table or counter top. As with the previous embodiment, the enclosure 2 comprises an outer shell portion 6 made of a suitable material, such as stainless steel. Located within outer shell portion 6 is an inner chamber 8 defined by inner partitions 10 and 12 made of, for example, stainless steel, into which transducer 4 may be inserted through an opening 40 in the outer shell 6. The transducer 4 is positioned on a loading platform 42 and is movable into and out of inner chamber 8 via a sliding load tray 44. Movement of sliding load tray 44 is preferably effected by push/pull handle 46. An ultraviolet lamp 16 (for example, a 9 watt UV lamp) and one or more reflecting surfaces 18 (e.g. mirrors, such as those described above) are located within inner chamber 8. If necessary for operation of the lamp 16, a ballast 20 may be included within shell portion 6.

Located on an outside surface of shell 6, for example, the front surface as shown in Figure 2(a), is a power on/off switch 22, a cycle start button 24 and a programmable or fixed exposure timer 26. Power is supplied to the enclosure 2 via power cord 28. Enclosure 2 may include mounting pads 48, made of rubber, for example, for positioning the enclosure on a horizontal surface. The enclosure 2 is preferably compact in size with typical dimensions being, with reference to Figure 2(a), a width of 9 inches and a height of 6 inches, and, with reference to Figure 2(c), a length or depth of 11 inches. Again, of course, the inventive enclosure is not to be limited by any particular size or dimension. The enclosure 2 should be appropriately sized as is necessary to accommodate the particular ultrasound transducer or range of different size transducers.

In addition to the programmable or fixed timer 26 that insures the desired duration of a dose of UV required for sterilization, a UV output monitor (to constantly monitor the lamp output and provide a signal for bulb replacement) and/or an electronic device, that measures the length of time bulbs have been in service and signals or disables the device when bulb replacement is required, may be included in the inventive device.

Once the transducer 4 is positioned in the inner chamber 8, the transducer should be exposed to the UV light for a sufficient period such that sterilization of the desired surfaces occurs. For example, the U.S. Food and Drug Administration recognizes exposure to a UV wavelength of 253.7 nanometer and a dose of 300,000 microwatt seconds per square centimeter for 5 minutes as adequate for sterilization (i.e. all known bacteria and viruses being destroyed). Of course, higher wavelengths, doses and/or exposure times are acceptable.

The face or "lens" of an ultrasound transducer used for transcutaneous (in contact with the skin) diagnostic or therapeutic ultrasound procedures is commonly fabricated from silicone, epoxy, polyurethane or plastic. These materials can be degraded by ultraviolet light. The UV tolerance of the lens material may be increased by the addition to the lens material during manufacturing of a UV inhibitor, such as titanium dioxide powder, and/or chemicals such as hydroxyphenylbenoztrriazole or metallic sterates which have been utilized as UV inhibitors in non-medical applications such as vinyl siding, paints or skin sunscreens.

The face or "lens" of the transducer must be clean and not contain dirt particles, oils or couplant (scanning gels) that will prevent complete UV coverage and full UV dose to the surface. The face or lens may be wiped with a rough surface cleaning paper towellette impregnated with an alkyl dimethyl benzl ammonium chloride (such as Hyamine® 3500 from Lonza Specialty Chemicals, Fair Lawn, NJ), which can reduce the surface tension and interfacial tension to under 33.0 dynes/centimeter, thus facilitating the removal of oils and dirt to allow complete UV coverage at full dose. In addition, at 200 ppm, alkyl dimethyl benzl ammonium chloride is an effective 30 second antimicrobial that further enhances the UV sterilization.

Although enclosure 2 has been described as having an inner chamber 8 located within outer shell portion 6, this is not a requirement of the inventive device. Inner chamber 8 is preferred to be included so as to isolate the lamp 18 and reflectors 18 from other internal components thus shielding the other components from any UV bombardment.

In operation, once a transducer 4 has been positioned in opening 14 (Figure 1(a) or 1(b)) or placed on sliding load tray 44 (Figure 2(b)) and inserted into chamber 8 (Figure 2(c)), power switch 22 is moved to the "on" position, the appropriate exposure time is entered into the exposure timer 26 and the cycle start button 24 is pushed. Once the cycle is complete, the transducer 4 may be removed and utilized in another ultrasound procedure. The amount of time programmed into the timer 26 will vary based on the desired dose of UV light, the intensity of the UV light and/or the distance from the UV light source to the transducer. Alternatively, the timer 26 can be of a fixed time length which delivers the minimum UV light dose needed for sterilization of the largest transducer that can be accommodated by the inner chamber 8.

The present invention provides for a fast, convenient, compact and neat sterilizing device and method for ultrasound transducers.

While the invention has been described with reference to preferred embodiments it is to be understood that the invention is not limited to the particulars thereof. The present invention is intended to include modifications which would be apparent to those skilled in the art to which the subject matter pertains without deviating from the spirit and scope of the appended claims.

## Claims

1. An apparatus for sterilizing one or more surfaces of an ultrasound transducer, said apparatus comprising:
a chamber comprising an opening through which an ultrasound transducer can be inserted into and withdrawn from the chamber;
at least one source of UV light capable of delivering a dose of ultraviolet wavelength (UV) light into said chamber, said dose being sufficient to sterilize one or more surfaces of the transducer.

2. The apparatus of claim 1 wherein said chamber includes one or more reflecting surfaces.

3. The apparatus of claim 2 wherein said one or more reflecting surfaces comprise mirrors.

4. The apparatus of claim 3 wherein said mirrors comprise one or both of coated metals and metalized plastics.

5. The apparatus of any one of the preceding claims wherein said source of UV light comprises a UV lamp.

6. The apparatus of any one of the preceding claims wherein said chamber includes means to secure in a releasable manner the one or more surfaces of the transducer within said chamber.

7. The apparatus of any one of the preceding claims further including a movable tray for transporting an ultrasound transducer toward and away from said chamber whereby the one or more surfaces of the transducer may be inserted into and withdrawn from said chamber via said opening.

8. The apparatus of any one of the preceding claims further including a programmable or fixed exposure timer for said source of UV light.

9. The apparatus of any one of the preceding claims further including a UV output monitor.

10. The apparatus of claim 9 wherein said output monitor includes a signal means for indicating the need to replace the source of UV light.

11. The apparatus of any one of the preceding claims including means to measure the length of time the source of UV light has been in service.

12. The apparatus of any one of the preceding claims further including a means to provide a signal indicative of when the UV light source has been in service for a predetermined amount of time.

13. The apparatus of any one of the preceding claims further including a means to disable said apparatus when the UV light source has been in service for a predetermined amount of time.

14. The apparatus of any one of the preceding claims wherein said chamber is located within an outer enclosure.

15. The apparatus of any one of the preceding claims including mounting surfaces whereby said apparatus is positionable on a generally horizontal surface.

16. The apparatus of any one of the preceding claims including means to mount said apparatus on a generally vertical surface.

17. The apparatus of any one of the preceding claims being positionable in a medical ultrasound system, an electronic system or a control cabinet.

18. A method of sterilizing one or more surfaces of an ultrasound transducer, said method comprising:
providing a chamber comprising an opening through which the ultrasound transducer can be inserted into and withdrawn from the chamber, said chamber including a source of UV light capable of delivering a dose of ultraviolet wavelength (UV) light into said chamber;
inserting the one or more surfaces of the ultrasound transducer into said chamber;
activating the UV light and exposing said one or more surfaces of the transducer to said UV light for a predetermined amount of time whereby said one or more surfaces of the transducer are sterilized;
withdrawing the sterilized transducer from the chamber.

19. The method of claim 18 wherein said chamber includes one or more reflecting surfaces.

20. The method of claim 18 or claim 19 wherein said one or more surfaces of the transducer comprises a front face and front sides of the transducer.

21. The method of claim 20 wherein the front face of the transducer includes a UV inhibitor.

22. The method of any one of claims 18 to 21 wherein said exposure is at least at a UV wavelength of 253.7 nanometer and a dose of 300,000 microwatt seconds per square centimeter for 5 minutes.

23. The method of any one of claims 18 to 22 further comprising cleaning said one or more surfaces of the ultrasound transducer prior to said exposure.

24. The method of any one of claims 18 to 23 wherein said ultrasound transducer is one of an ultrasound transducer utilized for imaging procedures, an ultrasound transducer utilized for therapeutic procedures, or, a high intensity focused ultrasound (HIFU) transducer.
